(19) Europäisches Patentamt
European Patent Office
Office européen des brevets

(11) Veröffentlichungsnummer: **0 030 213**
**A1**

(12) # EUROPÄISCHE PATENTANMELDUNG

(21) Anmeldenummer: 80810363.4

(22) Anmeldetag: 24.11.80

(51) Int. Cl.³: **G 03 F 7/10**
G 03 F 7/02, G 03 C 1/71
G 03 C 1/68, G 03 C 1/72

(30) Priorität: 29.11.79 CH 10602/79
25.04.80 CH 3206/80

(43) Veröffentlichungstag der Anmeldung:
10.06.81 Patentblatt 81/23

(84) Benannte Vertragsstaaten:
CH DE FR GB IT LI

(71) Anmelder: CIBA-GEIGY AG
Patentabteilung Postfach
CH-4002 Basel(CH)

(71) Anmelder: Hasek, Josef
Rosgartenstrasse 34
CH-8280 Kreuzlingen(CH)

(72) Erfinder: Hasek, Josef
Rosgartenstrasse 34
CH-8280 Kreuzlingen(CH)

(72) Erfinder: Zahir, Sheik Abdul-Cader, Dr.
Elsternstrasse 12
CH-4104 Oberwil(CH)

(72) Erfinder: Schneider, Wolfgang
Liebrütistrasse 24, 141
CH-4303 Kaiseraugst(CH)

(54) **Durch Licht vernetzbare Schicht auf Druckformen und Verfahren zur Herstellung von Offset-Druckplatten.**

(57) Die lichtempfindliche, homogene Schicht enthält beispielsweise als monomere oder oligomere, olefinisch ungesättigte lichtvernetzbare Verbindungen Epoxyacrylate und als Initiator und/oder Sensibilisator Benzildimethylketal in Kombination mit Michler's Keton. Die Schicht enthält zusätzlich keine Verbindungen, insbesondere Polymere, welche aus nicht UV-belichteten Bereichen entfernbar sind.

EP 0 030 213 A1

Durch Licht vernetzbare Schicht auf Druckformen und Verfahren zur Herstellung von Offset-Druckplatten.

Es sind bereits durch Licht vernetzbare Schichten und Massen bzw. Mischungen bekannt, bei welchen nach der Belichtung die nicht belichteten und somit unvernetzten Teile mittels wässriger, alkalischer Entwickler-Lösungen entfernt werden. Als besonders einschlägiger Stand der Technik ist hier die JP -Offenlegungsschrift Sho 49-44801 (27. April 1974) anzuführen, welche eine photoempfindliche Schicht für Druckformen und insbesondere eine entsprechende Herstellung von Offset-Druckplatten betrifft. Die photoempfindliche Schicht besteht im wesentlichen aus einem photopolymerisierbaren Epoxyacrylatharz und einem Copolymer, welches durch wässrig alkalische Lösungen unter Salzbildung löslich ist. Diese Copolymeren enthalten mindestens ein alkalisch-wässrig lösliches Comonomer einpolymerisiert. Als Beispiele für solche Copolymere werden in der JP -OS u.a. Copolymere von Styrol mit Maleinsäureanhydrid und Copolymere von Methylmethacrylat mit Maleinsäureanhydrid angeführt.

Weiter enthält die lichtempfindliche Schicht auch noch Photoinitiatoren, thermische Polymerisationsinhibitoren und gegebenenfalls Farbstoffe. Das in der Schicht enthaltene alkalisch-wässrig lösliche Copolymer ist während der Belichtung der Schicht mit dem Epoxyacrylat weiter copolymerisierbar. Das Gewichtsverhältnis dieses Copolymeren zu dem Epoxyacrylatharz beträgt 10:60 bis 10:40. Es wird ausdrücklich betont, dass die Anwendung geringerer Mengen als 10 Gewichtsteile des wasserlöslichen Copolymers sich nachteilig auf das Herstellungsverfahren der Offset-Druckplatten auswirkt. Die wässrige Entwicklung mit alkalischen Lösungen ist dann schwieriger durchführbar.

Weiterer Stand der Technik ist auch die DE-OS 2 843 762, welche eine Offsetdruckplatte betrifft. Letztere enthält als eigentliche photopolymerisierbare obere Schicht ein Gemisch aus verschiedenen Harzen, welche teilweise eine entsprechende Lichtempfindlichkeit aufweisen, Photoinitiatoren und ein wasserlösliches Celluloseharz. Ausserdem enthält die Offsetdruckplatte noch eine lichtempfindliche Zwischenschicht auf der Basis von, vorzugsweise wasserlöslichen, Diazoverbindungen. Auffallend ist, dass auch hier, wie bei der vorher besprochenen JP-Offenlegungsschrift, in der photopolymerisierbaren Schicht eine wasserlösliche Verbindung (das Celluloseharz) enthalten ist. Der Zweck ist offensichtlich auch hier die Verbesserung der Entwicklung auf wässriger Basis.

Es wurde nun gefunden, dass Schichten auf Druckformen, welche im wesentlichen lediglich aus einem Gemisch aus durch Licht vernetzbaren Epoxyacrylaten und bestimmten Photoinitiatoren bestehen und welche im Gegensatz zu den Systemen der JP-OS Sho 49-44801 und denen der DE-OS 2 843 762 überhaupt keine Copolymeren, welche unter Salzbildung wässrig-alkalisch auslösbar sind, oder wasserlösliche Celluloseharze enthalten, überraschend auch besonders gut für die Herstellung von Offset-Druckplatten, unter Anwendung wässriger Entwicklungsverfahren geeignet sind. Derartige Schichten sind sehr lichtempfindlich und liefern letztlich klare Bilder mit scharfen Konturen. Die Haftung des entsprechenden Films ist besonders gross. Derartige Schichten weisen eine gegenüber den Systemen des Standes der Technik verbesserte Langzeitlagerfähigkeit der fertigen Druckplatte auf. Die mögliche Lagerzeit beträgt ohne Anwendung von irgendwelchen Schutzschichten mehr als 6 Monate. Bei bekannten Systemen beträgt diese Zeit nur 2 Wochen, was zur Folge hat, dass hier Schutzschichten, wie beispielsweise PVA-Filme, angebracht werden müssen.

Das Auffinden dieser neuen Schichten auf Druckformen musste insbesondere deshalb ausserordentlich überraschen, da die oben erwähnte JP-OS lehrt, dass solche Schichten, in denen der angegebene minimale Anteil des alkalisch-wasserlöslichen Copolymeren unterschritten ist, praktisch unbrauchbar für Herstellungsverfahren von Offset-Druckplatten sind, bei denen wässrig-alkalisch entwickelt wird.

Gegenstand der Erfindung ist eine lichtvernetzbare, homogene Schicht auf Druckformen, welche nach der Photoanwendung wässrig entwickelbar ist und welche dadurch gekennzeichnet ist, dass sie

a) eine monomere oder oligomere, olefinisch ungesättigte, zu einem vernetzten Polymeren polymerisierbare Verbindung auf der Basis von mit Acryl- oder Methacrylsäure modifizierten Epoxidharzen mit einer Säurezahl von $< 0,2$, vorzugsweise $< 0,1$ Val/kg und

b) mindestens einen bei Bestrahlung anregbaren Initiator und/oder Sensibilisator in einer Menge von 0,1-30 Gew.-%, bezogen auf die Komponente a), enthält,

und dass die Schicht frei von Verbindungen, insbesondere von Polymeren, ist, welche aus nicht belichteten bzw. nicht vernetzten Bereichen der Schicht noch wässrig entfernbar sind.

Bevorzugt ist eine solche erfindungsgemässe, lichtvernetzbare Schicht, welche als Komponente a) eine monomere oder oligomere, olefinisch ungesättigte, durch freie Radikale oder durch Bestrahlung zu einem vernetzten Polymeren polymerisierbare Verbindung auf der Basis von mit Acryl- oder Methacrylsäure modifizierten Epoxidharzen enthält, die noch zusätzlich durch Zugabe von Maleinsäureanhydrid ($< 0.3$ mol per mol Epoxid im unmodifizierten Epoxidharz) so modifiziert wird, dass die Säurezahl $< 0.2$, vorzugsweise $< 0.1$ Val/kg, beträgt.

Besonders bevorzugt ist eine solche lichtvernetzbare homogene Schicht, welche die Komponenten a) und b), und

c) bis zu 50 Gew.-%, bezogen auf die Komponente a), einer photopolymerisierbaren Verbindung der Formel I

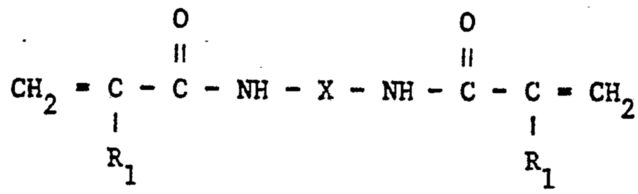 (I),

worin $R_1$ Wasserstoff oder Methyl und X eine Methylenkette mit 1 bis 12 C-Atomen, $-CH(CH_3)-$, $-C(CH_3)_2-$ oder $-CH(CCl_3-$ bedeuten und

d) gegebenenfalls weitere Hilfsstoffe, wie Epoxid-Novolak-Harze Polymerisationsinhibitoren, Farbstoffe, Verlaufmittel und dergleichen enthält.

Von besonderem Interesse als Komponente c) sind photopolymerisierbare Verbindungen der Formel I, worin $R_1$ Wasserstoff und X Methylen bedeuten.

Vorteilhafterweise setzt man 10-30 Gew.-% der Komponente c), bezogen auf die Komponente a), ein. Der Zusatz bewirkt eine raschere Polymerisation und verleiht der Schicht bessere mechanische Eigenschaften.

Vorteilhafterweise enthält die erfindungsgemässe Schicht als Komponente d) ein Epoxid-Novolak-Harz in einer Menge von bis zu 50 Gew.-%, bezogen auf die Komponente a).

Technisch besonders günstig ist auch eine lichtvernetzbare, erfindungsgemässe Schicht, welche als die unter a) genannte, ungesättigte Verbindung ein Reaktionsprodukt aus einem Epoxidharz und Acrylsäure oder Methacrylsäure, welches durch Reaktion mit einer Verbindung aus der Gruppe Crotonsäure, Sorbinsäure und Monoaddukt aus Maleinsäure- oder Phthalsäureanhydrid mit Hydroxyalkylacrylat oder -methacrylat modifiziert worden ist, enthält, wobei das Mol-Verhältnis der Acryl- oder Methacrylsäure zu der für die Modifizierung verwendeten Verbindung $\frac{500}{1}$ bis $\frac{0,05}{1}$, vorzugsweise $\frac{4}{1}$, beträgt.

Eine weitere Vorzugsform der Erfindung ist eine lichtvernetzbare Schicht, welche dadurch gekennzeichnet ist, dass sie als die unter a) genannte, ungesättigte Verbindung ein Epoxyacrylat oder -methacrylat enthält, welches noch freie Epoxidgruppen aufweist, und dass die Schicht zusätzlich Härter und gegebenenfalls Beschleuniger für Epoxidharze enthält.

Geeignete bei Bestrahlung anregbare Initiatoren, wie sie unter b) angeführt werden, sind z.B. folgende Verbindungen: Benzoine, wie Benzoin, Benzoinmethyläther, Benzoinäthyläther, Benzoinisobutyläther, Benzoinisopropyläther, α-Methylbenzoin, α-Aethylbenzoin, α-Phenylbenzoin, α-Allylbenzoin; Anthrachinone, wie Anthrachinon, Chloranthrachinon, Methylanthrachinon, Aethylanthrachinon, Diketone, wie Benzil, Benzilketale, wie Benzildimethylketal, Diacetyl, 2,2-Dialkoxysubstituierte Acetophenone, wie 2,2-Diäthoxy-acetophenon; 2,2-Dialkoxy-2-phenyl-substituierte Acetophenone, wie 2,2-Dimethoxy-2-phenylacetophenon; Disulfide, wie Diphenyldisulfid, Tetraäthylthiuramdisulfid; 2-Naphthalin-sulfonylchlorid. Grundsätzlich kann erfindungsgemäss auch ein Gemisch mehrerer Initiatoren und/oder mehrerer Sensibilisatoren eingesetzt werden.

Bevorzugt enthält die erfindungsgemässe Schicht als den unter b) genannten Initiator und/oder Sensibilisator Benzildimethylketal in Kombination mit Michler's Keton im Molverhältnis $\frac{100}{1}$ bis $\frac{1}{1}$, vorzugsweise $\frac{5}{1}$ bis $\frac{2}{1}$.

Zu den gegebenenfalls zusetzbaren unter d) genannten Hilfsstoffen ist folgendes näher auszuführen. Durch die Zugabe von bis zu 50 Gew.-%, insbesondere von etwa 10 Gew.-% eines Epoxid-Novolak-Harzes, bezogen auf die Komponente a), erhält die erfindungsgemässe Schicht eine ausgezeichnete Säurebeständigkeit. Geeignete Polymerisationsinhibitoren, die bis zu 1 Gew.-%, bezogen auf die Komponente a), zugegeben werden, sind beispielsweise 4-Methoxyphenol,

2,6-Di-tert.-butyl-p-kresol, Hydrochinone, 2,5-Di-tert.-butyl-hydrochinon und substituiertes oder unsubstituiertes Phenothiazin. Beispiele für in der erfindungsgemässen Schicht verwendbare Farbstoffe, die in einer Menge von 1-4 Gew.-%, vorzugsweise 2 Gew.-%, bezogen auf die Komponente a), eingesetzt werden, sind Bengalrosa, Brillantgrün, Kristallviolett und Rhodamin. Als geeignete Verlaufmittel, die bis zu 5 Gew.-%, bezogen auf die Komponente a), verwendet werden, sind insbesondere nichtionogene Benetzungsmittel anzuführen. Bekannte Handelsnamen für Verlaufmittel sind Antarox®, Gafen® und Solvophen®, BYK®, sowie fluorierte Kohlenwasserstoffe.

Als Hilfsstoffe besonderer Art sind lichtempfindliche, d.h. photohärtbare Diazoverbindungen herauszustellen. Es handelt sich im wesentlichen um folgende Verbindungen: o- oder p-Chinondiazidester oder -amide von Sulfon- oder Carbonsäuren, o-Diazoanisidin, Diazostilben, das tetraazotierte Di-o-anisidin-Zink-Doppelsalz und Kondensationsprodukte aus einem p-Diazidodiphenylamin und einer aktiven Carbonylverbindung. Zu diesen Produkten zählen Säure-Kondensationsprodukte eines Diazoniumsalzes von 4-Amino-1,1'-diphenylamin, z.B. 1,1'-Diphenylamin-4-diazoniumhalogenid oder 1,1'-Diphenylamin-4-diazoniumphosphat, kondensiert in Gegenwart einer Säure mit Aldehyden, z.B. Paraformaldehyd oder Formaldehyd, die in ein Doppelsalz überführt werden, beispielsweise ein Chlorzinkat oder Fluorborat. Bei diesen erfindungsgemäss verwendbaren Harzen handelt es sich um übliche bekannte Harze, deren Herstellung bekannt ist, beispielsweise aus dem Buch von Kosar, Light-Sensitive Systems, (New York 1965), Seiten 323 bis 324 und aus der US-PS 3 235 384.

Der Effekt der Einwirkung von Licht auf lichtempfindliche Diazoharze ist bekannt. Dabei werden negativ arbeitende Bilder erhalten, d.h. bei einer bildweisen Exponierung einer derartigen Harzschicht erfolgt eine Quervernetzung unter Erzeugung eines in Wasser unlöslichen Polymeren. Die nicht exponierten Bezirke des Originals bleiben vorzugsweise in Wasser löslich und sind abwaschbar, wenn sie mit einem geeigneten Lösungsmittel behandelt werden.

Diese Diazoverbindungen sind teilweise zwar auch wasserlösliche Substanzen. Die zugesetzten Mengen beschränken sich jedoch auf maximal 3 Gew.-%, bezogen auf die gesamte lichtvernetzbare Schicht. Bei der Herstellung der erfindungsgemässen Schicht erfolgt der Zusatz der Diazoverbindungen in Form einer Wasser enthaltenden Lösung zu der Ausgangslösung für den Beschichtungsvorgang.

Die zugesetzten Diazoverbindungen führen zu folgenden Verbesserungen. Sie bewirken eine vollständigere Vernetzung der Schicht durch die UV-Belichtung gegenüber lichtempfindlichen Schichten, bei denen dieser Hilfsstoff fehlt. Dies hat wiederum zur Folge, dass die Oberfläche nicht "aktiv" ist und dass die Farbaufnahme sehr gut ist. Da bei der Belichtung der Diazokörper ein Farbwechsel auftritt, erübrigt sich in der Schicht ein zusätzlicher Farbstoff.

Eine erfindungsgemässe, lichtvernetzbare Schicht, welche eine oder mehrere der beschriebenen Diazoverbindungen als Hilfsstoff in einer Konzentration bis zu 3 Gew.-%, bezogen auf die Komponente a), enthält, stellt eine weitere Vorzugsform der Erfindung dar.

Die Druckformen, auf denen sich die erfindungsgemässen, lichtempfindlichen Schichten befinden, sind insbesondere Zylinder oder Platten bzw. Bahnen aus Aluminium, Zink, Kupfer, Chrom oder Magnesium. Bevorzugt sind es Metallplatten, welche für den Offset-Druck geeignet sind. Grundsätzlich können es auch mehrschichtige Platten aus verschiedenen Metallen sein. Die Metalle sind vorzugsweise chemisch, elektrisch oder mechanisch vorbehandelt. So sind dieselben meistens oberflächlich chemisch oder anodisch oxidiert und vielfach mechanisch aufgerauht, um die Haftung der erfindungsgemässen Schicht zu optimieren. Geeignet für die Druckformen sind auch autotypischer Tiefdruck, Siebdruck sowie Klischee aus Zink oder Magnesium und Hochdruckreliefplatten.

Die Träger der lichtempfindlichen Schicht können aber auch Papierbahnen sein, welche vorzugsweise mit Copolymeren aus Vinyläthern und Maleinsäureanhydrid (z.B. mit Gantrez®) oder mit Polyvinylalkohol imprägniert sind. Grundsätzlich sind auch Polyester- oder Polyamidfolien als Träger anwendbar.

Die erfindungsgemässen, lichtempfindlichen Schichten sind empfindlich gegen UV-Licht, insbesondere von Wellenlängen zwischen 280 und 500 nm. Die optimale Empfindlichkeit liegt bei etwa 350 nm.

Weiterer Gegenstand der Erfindung ist ein Verfahren zur Herstellung von Offset-Druckplatten, welches dadurch gekennzeichnet ist, dass man

1. eine vorzugsweise auf der Oberfläche vorbehandelte Metallplatte mit einer 1- bis 30-gewichtsprozentigen Lösung einer lichtvernetzbaren Mischung, welche die Zusammensetzung der erfindungsgemässen lichtvernetzbaren Schicht aufweist, in einem organischen Lösungsmittel, vorzugsweise Glykolmonoäthyläther, in einer solchen Menge beschichtet, dass letztlich nach Entfernung des Lösungsmittels eine Schichtstärke von 1 bis 3 µm resultiert,

2. den so erhaltenen Film bei Temperaturen zwischen 40 und 100°C trocknet,

3. nach Aufbringung eines Negativfilms auf die Platte mittels einer UV-Lampe belichtet, und

4. abschliessend nach Entfernung des Negativfilms aus der teilweise belichteten Platte die Schicht mittels eines wässrigen Entwicklers mit einem pH-Wert bis 14, vorzugsweise 1 bis 13,5, entwickelt und die Oberfläche trocknet.

Bei dem erfindungsgemässen Verfahren kann die Beschichtung der Metallplatte mit der Lösung der jeweiligen lichtvernetzbaren Masse nach verschiedenen Verfahren erfolgen, beispielsweise durch Tauchen, mittels Walzen,durch Vorhanggiessen, durch elektrostatisches Auftragen und mittels einer Zentrifuge.

Als wässriger Entwickler kann eine Lösung einer oder mehrerer alkalischer Verbindungen mit einem pH zwischen 8 und 13 eingesetzt werden. Geeignete wässrig-alkalische Entwickler sind Lösungen von Na-, Li-, K- und Ca-Verbindungen, so z.B. von NaOH, Na-Silikaten, wie $Na_2SiO_3 \cdot 5H_2O$, Na-Azetaten, $Na_3PO_4 \cdot 12H_2O$, aber auch von Aminen, wie Aethanolamin.

Erfindungsgemäss können überraschend auch saure, wässrige Entwickler eingesetzt werden. So beispielsweise wässrige Lösungen von Phosphorsäuren, Salzsäure, Schwefelsäure, Salpetersäure, Ammoniumphosphat, Ammoniumnitrat, Aluminiumnitrat usw. Bevorzugt wird hier eine 0,5 bis 5%ige $H_3PO_4$-Lösung.

Selbstverständlich sind auch bei den sauren Entwicklern Lösungen von entsprechenden Mischungen der aufgezählten Verbindungen verwendbar.

Enthält die erfindungsgemässe, lichtvernetzbare Schicht als Hilfsstoff eines der beschriebenen Kondensationsprodukte auf der Basis p-Diazidodiphenylamin und einer aktiven Carbonylverbindung, so kann nach der Belichtung nur mittels wässriger Säure (z.B. 1%ige $H_3PO_4$) entwickelt werden. Bei den übrigen 3 Typen von Diazoverbindungen kann sowohl wässrig-alkalisch als auch wässrig-sauer entwickelt werden.

Beispiele zur Herstellung einzelner Komponenten.

I. Herstellung von durch Licht vernetzbaren Epoxyacrylaten oder -methacrylaten

Harz A: 2 kg eines flüssigen Bisphenol A-diglycidyläther-Harzes (Epoxidgehalt: 5,27 Val/kg) werden in einen 4-Hals-Glaskolben mit Rührer, Thermometer, Rückflusskühler und Tropftrichter gegeben. Mittels Oelbades wird das Harz unter Rührung auf 80°C erhitzt. Es werden 1,69 g Phenothiazin und 4,57 g Benzyl-trimethyl-ammoniumchlorid zugegeben. Die Erwärmung wird fortgesetzt, bis eine Temperatur von 110°C erreicht ist. Innerhalb von 1 1/2 Stunden werden aus dem Tropftrichter bei 110°C 722 g Acrylsäure langsam zugegeben. Das Rühren bei 110°C wird solange fortgesetzt, bis eine Säurezahl von 0,11 Val/kg erreicht wird. Danach wird die Mischung unter Rührung auf 100°C abgekühlt. 680 g Glykol-monoäthyläther werden zugegeben. Es entsteht so eine 80%ige Harzlösung, welche einen Epoxidgehalt von 0,2 Val/kg und eine Säurezahl von 0,07 Val/kg aufweist.

Harz B: Es wird das gleiche Harz A nach obigem Rezept hergestellt. Nach Zugabe des Lösungsmittels Glykol-monoäthyläther am Ende der Reaktion werden 21 g (0,14 Val/kg) Triäthanolamin zwecks Neutralisation von restlichen Acrylsäuregruppen zugegeben. Die so entstandene Lösung des Harzes B weist eine Säurezahl von weniger als 0,07 Val/kg auf.

Harz C: Es wird wie bei dem Verfahren zur Herstellung des Harzes A verfahren, nur mit folgenden Unterschieden. Es werden 570 g Acrylsäure, 1,6 g Phenothiazin und 3,6 g Benzyltrimethyl-ammoniumchlorid zugegeben. 4 1/2 Stunden nach Zugabe der Acrylsäure ergibt sich ein Gemisch, das eine Säurezahl von 0,1 Val/kg aufweist. Nach Kühlung auf 90°C werden 642 g Glykol-monoäthyläther zugegeben. Die so entstandene 80%-ige Harzlösung weist einen Epoxidgehalt von 0,69 Val/kg und eine Säurezahl von 0,001 Val/kg auf.

Harz D: 500 g eines flüssigen Bisphenol F-Epoxidharzes (Epoxidgehalt 6,06 Val/kg) werden gemäss dem Verfahren zur Herstellung des Harzes A mit 213 g Acrylsäure umgesetzt. Der Gehalt der Mischung an dem zugesetzten Phenothiazin beträgt 0,715 g, der an Benzyl-trimethyl-ammoniumchlorid 1,345 g. Die Zugabe der Acrylsäure bei 110°C dauert 1 1/2 Stunden. Danach wird während 30 Min. bei 110°C weitergerührt. Dann wird die Temperatur auf 115°C erhöht und die Mischung während 4 1/2 Stunden weitergerührt. Es ergibt sich eine Säurezahl von 0,16 Val/kg. Nach Kühlung weist das hochviskose Harz einen Epoxidwert von 0,14 Val/kg und nunmehr eine Säurezahl von 0,10 Val/kg auf.

Harz E: Es wird nach dem Verfahren zur Herstellung des Harzes A verfahren, nur mit folgenden Unterschieden: 2,5 kg eines flüssigen Epoxidharzes (Epoxidgehalt 7,3 Val/kg), hergestellt durch Umsetzung von Neopentylglykol mit Epichlorhydrin, werden mit 722 g Acrylsäure umgesetzt. Die Menge an Phenothiazin beträgt 3,78 g, die an Benzyltrimethylammoniumchlorid 8,12 g. Die Reaktion ist bei Erreichung einer Säurezahl von 0,16 Val/kg beendet. Nach Kühlung des Produktes weist dieses einen Epoxidgehalt von 0,24 Val/kg und eine Säurezahl von 0,03 Val/kg auf. Das Produkt ist niedrig-viskos; Viskosität: 1,25 Pa s bei 25°C.

Harz F: 136,6 g eines Epoxidharzes, hergestellt durch Umsetzung von p-Hydroxybenzoesäure mit Epichlorhydrin im Ueberschuss, mit einem Epoxidwert von 7,32 Val/kg werden nach dem Verfahren zur Herstellung von Harz A mit 72 g Acrylsäure umgesetzt. Die zugegebene Menge an Phenothiazin beträgt 0,104 g, die an Benzyl-trimethyl-ammoniumchlorid 0,45 g. Die Reaktion erfolgt bei 100°C solange, bis die Mischung eine Säurezahl von 0,15 Val/kg aufweist. Nach Kühlung auf Raumtemperatur weist das Produkt einen Epoxidgehalt von 0,08 Val/kg und eine Säurezahl von 0,06 Val/kg auf. Die Viskosität bei 40°C beträgt 13 Pa s.

Harz G: 500 g des zur Herstellung des Harzes E verwendeten Epoxidharzes auf Neopentylglykol-Basis werden mit 200 g Bisphenol A versetzt. Das Reaktionsgemisch wird auf 80°C erhitzt, worauf 0,05 g 2-Phenylimidazol zugegeben werden. Die Temperatur der so erhaltenen klaren Lösung wird langsam auf 130°C erhöht; sie wird während 45 Min. auf dieser Temperatur gehalten. Durch die Reaktion ergibt sich ein Epoxidwert von 2,75 Val/kg. Nach Kühlung der Mischung auf 110°C werden dieser 0,83 g Phenothiazin und 0,82 g Benzyl-trimethylammoniumchlorid und innerhalb von 1 Stunde 130 g Acrylsäure zugegeben. Danach wird die Temperatur der Mischung auf 115°C erhöht und die Umsetzung bei 115°C während 4 1/2 Stunden weitergeführt. Die Mischung weist dann eine Säurezahl von 0,18 Val/kg auf. Nach Zugabe von 205 g Glykol-monoäthyläther und Kühlung auf Raumtemperatur ergibt sich eine 80%-ige Lösung des Harzes G. Der Epoxidwert dieser Lösung beträgt 0,10 Val/kg, die Säurezahl 0,10 Val/kg.

Harz H: 560 g eines flüssigen Bisphenol A-Epoxidharzes (Epoxidgehalt 5,13 Val/kg) und 25 g eines festen Bisphenol A-Epoxidharzes (Epoxidgehalt 1,86 Val/kg) werden in einem 4-Hals-Glaskolben auf 110°C erwärmt und gerührt, bis eine homogene Lösung erhalten wird. Dieser werden 0,35 g Phenothiazin und 1,13 g Benzyl-triäthyl-ammoniumchlorid und danach bei 110°C innerhalb von 1 1/2 Stunden 179 g Acrylsäure zugegeben. Danach läuft die Reaktion noch weitere 2 Stunden bei 110°C, und dann noch weitere 3 Stunden bei 115°C. Es ergibt sich so eine Mischung mit einer Säurezahl von 0,06 Val/kg. Nach Zugabe von 176 g Glykol-monoäthyläther liegt eine 80%-ige Lösung des Harzes H vor. Diese Lösung weist einen Epoxidgehalt von 0,14 Val/kg und eine Säurezahl von 0,04 Val/kg auf.

Harz J: 500 g eines flüssigen Bisphenol A-Epoxidharzes (Epoxidgehalt 5,13 Val/kg) und 100 g eines festen Bisphenol A-Epoxidharzes (Epoxidgehalt 1,86 Val/kg) werden gemischt und gemäss dem Verfahren zur Herstellung des Harzes H mit 188,5 g Acrylsäure zur Reaktion gebracht.

Nach Erreichen einer Säurezahl von 0,11 Val/kg wird die Mischung mit 197 g Glykolmonoäthyläther verdünnt. Die so erhaltene 80%-ige Harzlösung weist nach Kühlung auf Raumtemperatur einen Epoxidwert von 0,15 Val/kg und eine Säurezahl von 0,06 Val/kg auf.

HARZ K:

1000 g eines flüssigen Bisphenol A-diglycidylätherharzes (Epoxidgehalt: 5,23 Val/kg) werden in einen 4-Hals-Glaskolben mit Rührer, Thermometer, Rückflusskühler und Tropftrichter gegeben. Mittels Oelbad wird das Harz unter Rühren auf 80°C erhitzt. Es werden 0,67 g Phenothiazin und 2,03 g Benzyl-triäthyl-ammoniumchlorid zugegeben. Die Erwärmung wird fortgesetzt, bis die Mischung eine Temperatur von 110°C aufweist. Innerhalb von 1 1/2 Stunden werden aus dem Tropftrichter bei 110°C 321 g Acrylsäure langsam zugegeben. Die Mischung wird bei 110°C solange gerührt, bis sie einen Säuregehalt von 0,2 Val/kg aufweist. Danach werden ihr 25,5 g Maleinsäureanhydrid zugegeben. Die Reaktion wird bei 110°C weitergeführt, bis ein Säuregehalt von 0,07 Val/kg erreicht ist. Nach dem Abkühlen auf etwa 100°C werden der Mischung 337 g Glykolmonoäthyläther zugegeben. Es entsteht so eine 80 %ige Harzlösung, welche einen Epoxidgehalt von 0,29 Val/kg und einen Säuregehalt von 0,03 Val/kg aufweist.

HARZ L:

1000 g eines flüssigen Bisphenol A-diglycidylätherharzes (Epoxidgehalt: 5,23 Val/kg) werden in einen 4-Hals-Glaskolben mit Rührer, Thermometer, Rückflusskühler und Tropftrichter gegeben. Mittels Oelbad wird das Harz unter Rühren auf 80°C erhitzt, worauf ihm 0,67 g Phenothiazin und 2,03 g Benzyl-triäthyl-ammoniumchlorid zugegeben werden. Die Erwärmung der Mischung wird fortgesetzt, bis eine Temperatur von 110°C erreicht ist. Innerhalb von 1 1/2 Stunden werden der Mischung aus dem Tropftrichter bei 110°C 321 g Acrylsäure langsam zugegeben. Die Mischung wird bei 110°C wird solange gerührt, bis ein Säuregehalt von

0,2 Val/kg erreicht wird. Danach werden ihr 51,3 g Maleinsäureanhydrid zugegeben. Die Reaktion wird bei 110°C weitergeführt, bis ein Säuregehalt von 0,07 Val/kg erreicht ist. Nach dem Abkühlen der Mischung auf etwa 100° werden dieser 343 g Aethylenglykolmonoäthyläther zugegeben. Es entsteht so eine 80%ige Harzlösung, welche einen Epoxidgehalt von 0,20 Val/kg und einen Säuregehalt von 0,04 Val/kg aufweist.

HARZ M:

1000 g eines Epoxykresolnovolakes mit einem Epoxidgehalt von 4,70 Val/kg werden in gleicher Weise wie bei Harz A beschrieben mit 1,07 g Benzyl-trimethyl-ammoniumchlorid und 5,85 g Phenothiazin versetzt und hierauf während 6 Stunden bei 110°C mit 170 g Acrylsäure reagieren gelassen. Nach dem Abkühlen gibt man zu der Mischung 294 g Butylacetat. Die erhaltene Lösung weist einen Epoxidgehalt von 1,36 Val/kg und einen Säuregehalt von 0,01 Val/kg auf.

HARZ N:

In gleicher Weise wie vorstehend beschrieben werden 500 g eines Phenolnovolakes mit einem Epoxidgehalt von 5,56 Val/kg mit 1,27 g Benzyl-trimethyl-ammoniumchlorid und 3,50 g Phenothiazin und hierauf mit 201 g Acrylsäure gemischt und während 9 Stunden bei 110°C reagieren gelassen.

Man erhält nach Zufügen von 176 g Butylacetat eine Harzlösung, die einen Epoxidgehalt von 0,3 Val/kg und einen Säuregehalt von 0,19 Val/kg aufweist.

HARZ O:

In gleicher Weise wie vorstehend beschrieben werden 1000 g eines Epoxyphenolnovolakes mit einem Epoxidgehalt von 5,62 Val/kg mit 1,92 g Benzyl-trimethyl-ammoniumchlorid und 0,65 g Phenothiazin

gemischt und hierauf mit 304 g Acrylsäure während 4,5 Stunden bei 110°C reagieren gelassen. Die nach Zugabe von 326 g Butylacetat erhaltene Harzlösung weist einen Epoxidgehalt von 0,85 Val/kg und einen Säuregehalt von 0,03 Val/kg auf.

HARZ P:

666 g Harz K werden mit

267 g flüssigem Epoxynovolak auf Phenolbasis (Epoxidgehalt 5,7 Val/kg, Viskosität bei 52°C: 1,4-2,0 Pas)

und 67 g Butylacetat vermischt.

## II. Wässrige Entwickler für das erfindungsgemässe Verfahren.

Entwickler a: 1%-ige $H_3PO_4$

Entwickler b: Bei der Herstellung geht man von einer Grundlösung aus, welche wie folgt hergestellt wird: 140 g NaOH, 140 g $Na_2SiO_3 \cdot 5H_2O$, 32 g $Na_3PO_4 \cdot 12H_2O$ und 32 g Na-Polyphosphat werden in Wasser bis zu einem Gesamtvolumen von 10 l gelöst. Der pH-Wert der so entstandenen Lösung liegt bei 13. Diese Grundlösung wird im Verhältnis 1:3 mit Wasser verdünnt. Die so verdünnte Lösung wird als Entwickler b bezeichnet.

## III. Erfindungsgemässe lichtvernetzbare Schichten auf Druckformen und Herstellung von Offset-Druckplatten.

Beispiele 1 bis 25: Die Beispiele sind in der nachfolgenden Tabelle veranschaulicht. Bei den Versuchen wird jeweils wie folgt verfahren. Nach Herstellung der gewünschten Lösung des durch Licht vernetzbaren Epoxyacrylates werden verschiedene Mengen von Photoinitiatoren und vernetzbarem Comomomeren (Methylen-bis-acrylamid) zugegeben.

In den Beispielen 22-25 werden diese in 500 g Isopropanol und 360 g Methäthylketon gelöst zugegeben. Oberflächlich oxidierte, aber sonst nicht vorbehandelte Aluminiumplatten werden in einer waagrecht laufenden Zentrifuge, welche eine Temperatur von 45°C aufweist, in waagrechter Anordnung befestigt. Unter Rotation wird die jeweilige Harzlösung zentral auf die Platte gegeben. Es wird mit 70 Umdrehungen pro Minute bei 45°C zentrifugiert bis eine trockene, nicht klebrige Oberfläche vorliegt. Die so beschichtete Al-Platte wird mit einem Negativfilm und am Rande mit einem UGRA-Offset-Testkeil (Erklärung unten) bedeckt und in einem Vakuum-Rahmen untergebracht. Zum Zwecke optimalen Kontaktes zwischen Al-Platte und Negativfilm wird Vakuum angelegt. Im Abstand von 120 cm wird mit einer 5000 W Metallhalogenid-Lampe 25 bis 60 Sek. lang belichtet. Danach wird die jeweilige teilweise belichtete Platte mittels der wässrigen Entwickler a oder b entwickelt. Es wird die erforderliche Mindest-Entwicklungszeit in Sek. gemessen. Nach Fertigstellung der jeweiligen Offset-Druckplatte wird dieselbe einem Test der UGRA (Verein zur Förderung wissenschaftlicher Untersuchungen in graphischen Gewerbe EMPA-C, Postfach 977, CH-9001 St. Gallen) unter Anwendung des "UGRA-Offset-Testkeils" unterworfen. Die Methode ist in einer Technischen Beschreibung dieses Vereins genauer erläutert. Im vorliegenden Falle kommt der Halbton-Keil zur Anwendung. Die verhältnismässig hohen Werte der jeweiligen Stufe (siehe die Tabelle) deuten auf eine hohe Empfindlichkeit hin.

Tabelle

| Beispiel | Ausgangslösung des durch Licht vernetzbaren Epoxyacrylates | | | Photoinitiatoren | | Bestrahlung mit UV-Licht (5000 W-Metallhalogenid-Lampe) Sek. | Entwickler und Mindest-Entwicklungszeit | | Halbton bei Anwendung des UGRA-Testkeils | Beurteilung der Offset-Druckplatte |
|---|---|---|---|---|---|---|---|---|---|---|
| | Harz-Typ Gew.-% | Lösungsmittel | Konzentration Gew.-% | Benzildimethylketal Gew.-% bezogen auf Epoxyacrylat | Michler's Keton Gew.-% bezogen auf Epoxyacrylat | | a Sek. | b Sek. | Stufe | |
| 1 | A 100 | Glykol-mono-äthyläther | 4 | 10 | 2 | 30 | 10 | | 8 | Gutes optisches Auflösungsvermögen, ausgezeichnetes Farbaufnahmevermögen |
| | | | | | | 60 | 10 | | 10 | " |
| 2 | A 100 | " | 4 | 10 | 2 | 30 | | 15 | 8 | " |
| | | | | | | 60 | | 15 | 10 | " |
| 3 | B 100 | " | 4 | 10 | 2 | 30 | 10 | | 8 | " |
| | | | | | | 60 | 10 | | 9 | " |
| 4 | B 100 | " | 4 | 10 | 2 | 30 | | 10 | 8 | " |
| | | | | | | 60 | | 10 | 9 | " |
| 5 | C 100 | " | 4 | 10 | 2 | 30 | 15 | | 6 | " |
| | | | | | | 60 | 15 | | 8 | " |

Tabelle (Forts.)

| Beispiel | Ausgangslösung des durch Licht vernetzbaren Epoxyacrylates | | | Photoinitiatoren | | Bestrahlung mit UV-Licht (5000 W-Metallhalogenid-Lampe) Sek. | Entwickler und Mindest-Entwicklungszeit | | Halbton bei Anwendung des UGRA-Testkeils | Beurteilung der Offset-Druckplatte |
|---|---|---|---|---|---|---|---|---|---|---|
| | Harz-Typ Gew.-% | Lösungsmittel | Konzentration Gew.-% | Benzildimethylketal Gew.-% bezogen auf Epoxyacrylat | Michler's Keton Gew.-% bezogen auf Epoxyacrylat | | a Sek. | b Sek. | Stufe | |
| 6 | | Glykolmonoäthyläther | 4 | 10 | 2 | 30 | | 15 | 6 | Gutes optisches Auflösungsvermögen, ausgezeichnetes Farbaufnahmevermögen |
| | | | | | | 60 | | 15 | 8 | " |
| 7 | E 100 | " | 5 | 10 | 5 | 30 | 4 | | 4/5 | " |
| 8 | E 100 | " | 5 | 10 | 5 | 60 | | 10 | 6 | " |
| 9 | F 100 | " | 5 | 10 | 5 | 45 | | 10 | 7 | " |
| 10 | 90 F und 10 E | " | 5 | 10 | 5 | 60 | | 10 | 6 | " |

Tabelle (Forts.)

| Beispiel | Ausgangslösung des durch Licht vernetzbaren Epoxyacrylates | | | Photoinitiatoren | | Bestrahlung mit UV-Licht (5000 W-Metallhalogenid-Lampe) | Entwickler und Mindest-Entwicklungszeit | | Halbton bei Anwendung des UGRA-Testkeils | Beurteilung der Offset-Druckplatte |
|---|---|---|---|---|---|---|---|---|---|---|
| | Harz-Typ Gew.-% | Lösungsmittel | Konzentration Gew.-% | Benzildimethylketal Gew.-% bezogen auf Epoxyacrylat | Michler's Keton Gew.-% bezogen auf Epoxyacrylat | Sek. | a Sek. | b Sek. | Stufe | |
| 11 | 90 A und 10 E | Glykolmonoäthyläther | 5 | 10 | 5 | 25 | | 10 | 8 | Gutes optisches Auflösungsvermögen, ausgezeichnetes Farbaufnahmevermögen |
| 12 | 90 D +10E | " | 5 | 10 | 5 | 30 | | 10 | 8 | " |
| 13 | II 100 | " | 5 | 10 | 5 | 30 | 12 | | 4/5 | " |
| 14 | II 100 | " | 5 | 10 | 5 | 30 | | 8 | 4/5 | " |
| 15 | II 100 | " | 5 | 10 | 5 | 60 | 12 | | 6 | " |

Tabelle (Forts.)

| Beispiel | Ausgangslösung des durch Licht vernetzbaren Epoxyacrylates | | | Photoinitiatoren | | Bestrahlung mit UV-Licht (5000 W-Metallhalogenid-Lampe) Sek. | Entwickler und Mindest-Entwicklungszeit | | Halbton bei Anwendung des UGRA-Testkeils | Beurteilung der Offset-Druckplatte |
|---|---|---|---|---|---|---|---|---|---|---|
| | Harz-Typ Gew.-% | Lösungsmittel | Konzentration Gew.-% | Benzildimethylketal Gew.-% bezogen auf Epoxyacrylat | Michler's Keton Gew.-% bezogen auf Epoxyacrylat | | a Sek. | b Sek. | Stufe | |
| 16 | I 100 | Glykolmonoäthyläther | 5 | 10 | 5 | 60 | 8 | | 4 | Gutes optisches Auflösungsvermögen, ausgezeichnetes Farbaufnahmevermögen |
| 17 | I 100 | " | 5 | 10 | 5 | 60 | | 5 | 4 | " |
| 18 | G 100 | " | 8 | 16 | 8 | 60 | | 10 | 6 | " |
| 19 | 90 G und 10 E | " | 5 | 10 | 5 | 60 | | 10 | 6 | " |

Tabelle (Forts.)

| Beispiel | Ausgangslösung des durch Licht vernetzbaren Epoxyacrylates | | | Photoinitiatoren | | Methylen-bis-acrylamid | Bestrahlung mit UV-Licht (5000 W-Metallhalogenid-Lampe) | Entwickler und Mindest-Entwicklungszeit | | Halbton bei Anwendung des UGRA-Testkeils | Beurteilung der Offset-Druckplatte |
|---|---|---|---|---|---|---|---|---|---|---|---|
| | Harztyp, Gew.-% | Lösungsmittel | Konzentration Gew.-% | Benzildimethylketal Gew.-% bezogen auf Epoxyacrylat | Michler's Keton Gew.-% bezogen auf Epoxyacrylat | | Sek. | a Sek. | b Sek. | Stufe | |
| 20 | K 125 | Glykolmonoäthyläther | 4 | 10 | 5 | 20 | 60 | | 45 | 6 | *sehr gute Farbaufnahme |
| 21 | L 125 | Glykolmonoäthyläther | 4 | 10 | 5 | 20 | 60 | | 45 | 3 | * gute Farbaufnahme |

*Gutes optisches Auflösungsvermögen

Tabelle (Forts.)

| Beispiel | Ausgangslösung des durch Licht vernetzbaren Epoxyacrylates | | | Photoinitiatoren | | Methylen-bis-acrylamid | Bestrahlung mit UV-Licht (5000 W-Metallhalogenid-Lampe) Sek. | Entwickler und Mindest-Entwicklungszeit | | Halbton bei Anwendung des UGRA-Testkeils | Beurteilung der Offset-Druckplatte |
|---|---|---|---|---|---|---|---|---|---|---|---|
| | Harztyp, Gew.-% | Lösungsmittel | Konzentration Gew.-% | Benzildimethylketal Gew.-% bezogen auf Epoxyacrylat | Michler's Keton Gew.-% bezogen auf Epoxyacrylat | | | a Sek. | b Sek. | Stufe | |
| 22 | P=125 | Butylacetat | 4 | 10 | 5 | 20 | 60 | | 30 | 8 | Tiefenpunkt-Ugrakeil gut, Hochlichter-Ugrakeil gut, gute Farbaufnahme, |
| 23 | M=125 | Butylacetat | 4 | 10 | 5 | 20 | 60 | | 30 | 4 | wie oben |
| 24 | N=125 | Butylacetat | 4 | 10 | 5 | 20 | 60 | | 30 | 8 | wie oben |
| 25 | O-125 | Butylacetat | 4 | 10 | 5 | 20 | 60 | | 30 | 4 | wie oben |

Patentansprüche

1. Eine lichtvernetzbare, homogene Schicht auf Druckformen, welche nach der Photoanwendung wässrig entwickelbar ist, dadurch gekennzeichnet, dass sie

a) eine monomere oder oligomere, olefinisch ungesättigte, zu einem vernetzten Polymeren polymerisierbare Verbindung auf der Basis von mit Acryl- oder Methacrylsäure modifizierten Epoxidharzen mit einer Säurezahl von $< 0,2$, vorzugsweise $< 0,1$ Val/kg, und

b) mindestens einen bei Bestrahlung anregbaren Initiator und/oder Sensibilisator in einer Menge von 0,1-30 Gew.-%,bezogen auf die Komponente a), enthält,

und dass die Schicht frei von Verbindungen, insbesondere von Polymeren, ist, welche aus nicht belichteten bzw. nicht vernetzten Bereichen der Schicht noch wässrig entfernbar sind.

2. Eine lichtvernetzbare homogene Schicht nach Anspruch 1, dadurch gekennzeichnet, dass sie als Komponente a) eine monomere oder oligomere, olefinisch ungesättigte, durch freie Radikale oder durch Bestrahlung zu einem vernetzten Polymeren polymerisierbare Verbindung auf der Basis von mit Acryl- oder Methacrylsäure modifizierten Epoxidharzen, enthält, die noch zusätzlich durch Zugabe von Maleinsäurenanhydrid ($< 0.3$ mol per mol Epoxid im unmodifizierten Epoxidharz) so modifiziert wird, dass die Säurezahl $< 0.2$, vorzugsweise $< 0.1$ Val/kg, beträgt.

3. Eine lichtvernetzbare homogene Schicht nach Anspruch 1, dadurch gekennzeichnet, dass sie die Komponenten a) und b) und

c) bis zu 50 Gew.-%, bezogen auf die Komponente a), einer photopolymerisierbaren Verbindung der Formel I

$$CH_2=\underset{\underset{R_1}{|}}{C}-\overset{\overset{O}{\|}}{C}-NH-X-NH-\overset{\overset{O}{\|}}{C}-\underset{\underset{R_1}{|}}{C}=CH_2 \qquad (I),$$

worin $R_1$ Wasserstoff oder Methyl und X eine Methylenkette mit 1 bis 12 C-Atomen, $-CH(CH_3)-$, $-C(CH_3)_2-$ oder $-CH(CCl_3)-$ bedeuten und

d) gegebenenfalls weitere Hilfsstoffe, wie Epoxid-Novolak-Harze, Polymerisationsinhibitoren, Farbstoffe, Verlaufmittel und dergleichen enthält.

4. Eine lichtvernetzbare homogene Schicht nach Anspruch 3, dadurch gekennzeichnet, dass sie als Komponente c) eine photopolymerisierbare Verbindung der Formel I, worin $R_1$ Wasserstoff und X Methylen bedeuten, enthält.

5. Eine lichtvernetzbare homogene Schicht nach Anspruch 3, dadurch gekennzeichnet, dass sie 10-30 Gew.-% der Komponente c), bezogen auf die Komponente a), enthält.

6. Eine lichtvernetzbare homogene Schicht nach Anspruch 3, dadurch gekennzeichnet, dass sie als einen unter d) genannten Hilfsstoffe ein Epoxid-Novolak-Harz in einer Menge von bis zu 50 Gew.-%, bezogen auf die Komponente a), enthält.

7. Eine lichtvernetzbare homogene Schicht nach Anspruch 3, dadurch gekennzeichnet, dass sie als einen der unter d) genannten Hilfsstoffe eine photohärtbare Diazoverbindung aus der Gruppe o- oder p-Chinondiazidester, oder -amide von Sulfon- oder Carbonsäuren, o-Diazoanisidin,

Diazostilben, tetraazotiertes Di-o-anisidin-Zink-Doppelsalz und Kondensationsprodukte aus einem p-Diazidodiphenylamin und einer aktiven Carbonylverbindung, in einer Menge von bis zu 3 Gew.-%, bezogen auf die Komponente a), enthält.

8. Eine lichtvernetzbare Schicht nach Anspruch 1, dadurch gekennzeichnet, dass sie als eine unter a) genannte, ungesättigte Verbindung ein Reaktionsprodukt aus einem Epoxidharz und Acrylsäure oder Methacrylsäure, welches durch Reaktion mit einer Verbindung aus der Gruppe Crotonsäure, Sorbinsäure und Monoaddukt aus Maleinsäure- oder Phthalsäureanhydrid mit Hydroxyalkylacrylat oder -methacrylat modifiziert worden ist, enthält, wobei das Mol-Verhältnis der Acryl- oder Methacrylsäure zu der für die Modifizierung verwendeten Verbindung $\frac{500}{1}$ bis $\frac{0,05}{1}$, vorzugsweise $\frac{4}{1}$, beträgt.

9. Eine lichtvernetzbare Schicht nach Anspruch 1, dadurch gekennzeichnet, dass sie als eine unter a) genannte, ungesättigte Verbindung ein Epoxyacrylat oder -methacrylat enthält, welches noch freie Epoxidgruppen aufweist, und dass die Schicht zusätzlich Härter und gegebenenfalls Beschleuniger für Epoxidharze enthält.

10. Eine lichtvernetzbare Schicht nach Anspruch 1, dadurch gekennzeichnet, dass sie als einen unter b) genannten Initiator und/oder Sensibilisator Benzildimethylketal in Kombination mit Michler's Keton im Molverhältnis $\frac{100}{1}$ bis $\frac{1}{1}$, vorzugsweise $\frac{5}{1}$ bis $\frac{2}{1}$, enthält.

11. Verfahren zur Herstellung von Offset-Druckplatten, dadurch gekennzeichnet, dass man

1.) eine vorzugsweise auf der Oberfläche vorbehandelte Metallplatte mit einer 1- bis 30-gewichtsprozentigen Lösung einer lichtvernetzbaren Mischung, welche die Zusammensetzung der lichtvernetzbaren Schicht nach Anspruch 1 aufweist, in einem organischen Lösungsmittel, in einer solchen Menge beschichtet, dass letztlich nach Entfernung des Lösungsmittels eine Schichtstärke von 1 bis 3 µm resultiert,

2.) den so erhaltenen Film bei Temperaturen zwischen 40 und 100°C trocknet,

3.) nach Aufbringung eines Negativfilms auf die Platte mittels einer UV-Lampe belichtet und

4.) abschliessend nach Entfernung des Negativfilms aus der teilweise belichteten Platte die Schicht mittels eines wässrigen Entwicklers mit einem pH-Wert bis 14, vorzugsweise 1 bis 13,5, entwickelt und die Oberfläche trocknet.

12. Verfahren nach Anspruch 11, dadurch gekennzeichnet, dass man als wässrigen Entwickler eine Lösung einer oder mehrerer alkalischer Verbindungen mit einem pH-Wert zwischen 8 und 13 einsetzt.

13. Verfahren nach Anspruch 11, dadurch gekennzeichnet, dass man als wässrigen Entwickler eine $H_3PO_4$-Lösung, vorzugsweise 0,5 bis 5%ig, einsetzt.

Europäisches Patentamt

# EUROPÄISCHER RECHERCHENBERICHT

Nummer der Anmeldung

EP 80810363.4

| EINSCHLÄGIGE DOKUMENTE | | | KLASSIFIKATION DER ANMELDUNG (Int Cl ) |
|---|---|---|---|
| Kategorie | Kennzeichnung des Dokuments mit Angabe, soweit erforderlich der maßgeblichen Teile | betrifft Anspruch | |
| X | DE - B - 1 104 339 (GESTETNER)<br>+ Gesamt +<br>--<br>DE - B - 1 597 782 (KALLE)<br>+ Gesamt +<br>--<br>DE - B - 2 207 209 (ASAHI)<br>+ Patentanspruch 5 +<br>---- | 1,11<br><br><br>1<br><br><br>1,3 | G 03 F 7/10<br>G 03 F 7/02<br>G 03 C 1/71<br>G 03 C 1/68<br>G 03 C 1/72<br><br>RECHERCHIERTE SACHGEBIETE (Int Cl )<br>G 03 F<br>G 03 C |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt

| Recherchenort | Abschlußdatum der Recherche | Prüfer |
|---|---|---|
| WIEN | 25-02-1981 | SALTEN |